# FASCICULE DE BREVET EUROPEEN

(11) **EP 0 502 055 B1**
(45) Date de publication et mention de la délivrance du brevet: **17.01.1996**
(21) Numéro de dépôt: 90917465.8
(22) Date de dépôt: 22.11.1990
(51) Int. Cl.: A61L 27/00

(54) **PROCEDE DE FABRICATION D'UN MATERIAU POUR OSTEOPLASTIE A PARTIR D'UN TISSU OSSEUX NATUREL ET MATERIAU OBTENU PAR CE PROCEDE**
VERFAHREN ZUR HERSTELLUNG EINES WERKSTOFFES FÜR OSTEOPLASTIK AUS NATÜRLICHEM KNOCHENGEWEBE SOWIE AUS SOLCHEM VERFAHREN GEWONNENES MATERIAL
METHOD OF MANUFACTURE OF A MATERIAL FOR OSTEOPLASTY FROM A NATURAL BONE TISSUE AND MATERIAL OBTAINED THEREBY

(30) Priorité: 22.11.1989 FR 8915363
(43) Date de publication de la demande: 09.09.1992
(73) Titulaire: OST-DEVELOPPEMENT, F-63100 Clermont-Ferrand (FR)
(72) Inventeur: BONIFACE, Robert, F-95810 Vallangoujard (FR); FAURIE, Michel, F-63960 Veyre-Monton (FR); GOLDSCHMIDT, Pablo, F-75004 Paris (FR); LONTRADE, Jean-Pierre, F-63000 Clermont-Ferrand (FR); LUYCKX, Jacques, F-63112 Ceyrat (FR)
(74) Mandataire: Thibon-Littaye, Annick
(86) Numéro de dépôt international: FR9000842
(87) Numéro de publication internationale: WO9107194

(56) Documents cités:
- FR-A- 1 317 584
- GB-A- 2 175 507
- GB-A- 2 175 807
- US-A- 4 440 750
- Proc. Natl. Acad. Sci. USA, volume 70, no. 12, Part I, décembre 1973, M.R.Urist et al.: "Bone morphogenesis in implants of insoluble bone gelatin", pages3511-3515

## Description

La présente invention concerne un matériau destiné à l'ostéoplastie ainsi qu'un procédé de mise en oeuvre pour son élaboration à partir de tissu osseux d'origine humaine pu animale. Elle vise essentiellement la mise à disposition des chirurgiens d'un matériau de substitution de l'os qui soit biocompatible, non-antigénique, ostéocompatible, ostéointégrable, etc.

Cette invention a pour but de produire des greffons, retaillables à la forme du défect osseux, permettant aux chirurgiens d'utiliser un tel greffon en tant qu'alternative soit à l'autogreffe, permettant ainsi d'éviter le deuxième site opératoire, soit à l'allogreffe, à cause des inconvénients et risques qu'elle représente.

Ce matériau est avantageusement amélioré par rapport à ceux de l'art antérieur de par son procédé de fabrication, particulièrement bien adapté pour en faire un greffon osseux en chirurgie réparatrice, traumatologique, orthopédique, maxillo-faciale ou mandibulaire ainsi qu'en chirurgie dentaire ou vétérinaire et, d'une façon générale, chaque fois qu'un site osseux fait appel à un greffon.

L'utilisation d'un tissu osseux de remplacement ou de substitution n'est pas une technique nouvelle. Les premières publications scientifiques datent des années 1800 et elles se sont depuis multipliées : elles concernent les allogreffes et les xénogreffes, dont les teneurs relatives en minéraux et en protéines peuvent varier selon les procédés. Parallèlement et postérieure-ment à l'emploi de ces substituts d'os d'origine humaine et animale, on a vu se développer l'utilisation de greffons de nature minérale : coraux, hydroxyapatites, céramiques, ou de nature synthétique comme les matériaux polymères biodégradables.

Aujourd'hui, les recherches entreprises dans ce domaine s'orientent vers l'emploi de matériaux plus proches de l'os dans leur structure et leur composition, et spécifiquement de l'os humain, afin de favoriser l'intégration du greffon osseux, qui passe par la reconnaissance par l'organisme de la nouvelle structure implantée et sa colonisation d'après le mécanisme dit de "substitution rampante".

L'os est un tissu vivant en perpétuel remaniement qui passe par la destruction et reconstruction simultanée de la matière osseuse. Dans le processus normal, mais aussi lorsqu'un matériau étranger est implanté, les ostéoclastes résorbent le tissu ancien, laissant place à une lacune dans laquelle les ostéoblastes élaborent le tissu nouveau. L'équilibre est toujours maintenu entre les quantités d'os résorbé et les quantités d'os nouvellement formé. Il apparaît que plus la structure implantée se rapproche de celle de l'os normal, plus ce phénomène va être facilité.

C'est ainsi que l'on préfère maintenant préparer des matériaux de substitution de l'os à partir de tissus osseux naturels, qui sont de préférence d'origine animale pour des raisons éthiques et pratiques.

D'une manière générale, on sait qu'une déminéralisation partielle favorise l'intégration d'une greffe osseuse. Celle-ci est donc suivie de différentes étapes complémentaires qui visent soit à déprotéiniser complètement l'os, soit à agir sur la nature des protéines qui restent alors liées dans la matrice osseuse, soit encore à augmenter cette teneur en protéines.

Parmi l'art antérieur on citera en particulier le brevet américain U.S. 4 394 370 qui envisage de fusionner en une masse spongieuse, au moyen d'un liant de glutaraldéhyde ayant un effet réticulant, un mélange constitué d'os pulvérulent déminéralisé d'origine humaine et de poudre de collagène reconstitué.

Le brevet américain U.S. 4 743 259 combine une déminéralisation par l'acide chlorhydrique avec un enrichissement en protéines effectué sur une première fraction de l'os déminéralisé, au moyen des protéines extraites d'une seconde fraction par la guanidine.

Par ailleurs, les demandes de brevets français publiées sous les numéros 2 582 517 et 2 582 518 proposent de traiter des fragments d'os prélevés sur des animaux, plus particulièrement sur des bovins, par déminéralisation partielle et tannage à l'aide de glutaraldéhyde. Les éléments osseux destinés à être implantés par le chirurgien sont découpés à la forme appropriée dans un os bovin auquel on a fait préalablement subir un traitement comportant une étape de délipidation ou dégraissage, par un solvant organique tel que l'éthanol, une étape de déminéralisation par un agent d'extraction du calcium tel que l'acide chlorhydrique, et une étape de tannage au glutaraldéhyde, ainsi que divers lavages.

La description de ces dernières demandes de brevets laisse entendre que ce tannage a une action bénéfique pour les propriétés de l'os traité par le fait qu'elle favorise une réticulation des chaînes macromoléculaires. Or il a été constaté maintenant qu'au contraire et à l'opposé des suggestions de l'art antérieur, le traitement au glutaraldéhyde n'entraîne pas de diminution sensible des propriétés immunogéniques, cependant que la réhabitation de l'os implanté n'est pas favorisée comme il est souhaitable pour assurer l'intégration du greffon osseux. De plus, des composés chimiques comme le glutaraldéhyde ont l'inconvénient d'être biologiquement toxiques.

A l'origine de la présente invention, on a pu observer qu'il est essentiel, pour obtenir une bonne intégration du greffon osseux, de conserver le collagène dans la matière osseuse, en le maintenant sous la forme spécifique qu'il présente dans l'os naturel, principalement en collagène de type I, tout en permettant de conserver l'aspect microscopique de l'os et de lui maintenir ainsi sa structure spatiale support d'une colonisation cellulaire ultérieure. On a pu observer également que les traitements de l'art antérieur ont sensiblement le même effet sur le collagène de l'os que sur les autres protéines qu'ils visent à éliminer ou à dénaturer pour s'affranchir de leur antigénicité.

L'invention a donc pour objet un procédé de traitement d'un tissu osseux d'origine humaine ou animale qui conduit à un matériau de substitution de l'os caractérisé par la conservation de la structure osseuse d'origine, intégrant du collagène de type I non dénaturé, mais débarrassée des protéines antigéniques. Elle résulte de la découverte surprenante qu'il est possible d'opérer sur les tissus osseux naturels une étape d'extraction sélective des protéines antigéniques en préservant le collagène de structure.

Plus précisément, le procédé suivant l'invention est caractérisé en ce qu'il comporte au moins une étape d'extraction et/ou dénaturation sélective des protéines de structure non collagéniques, réalisée au moyen d'un agent d'extraction sélectif à base d'urée. L'urée est utilisée avantageusement, dans le cadre de la présente invention, sous forme d'une solution aqueuse, sous une concentration généralement comprise entre 2 et 10 M, et de préférence entre 5 et 8 M. La température est la température ordinaire, ou en général une température comprise entre 15 et 30 °C.

Le suivi analytique du procédé selon l'invention, impliquant la mise en oeuvre de l'urée ou d'un agent d'extraction analogue, montre que l'on provoque ainsi des réactions tendant à rompre les chaînes macromoléculaires des protéines indésirables qui subsistent dans la structure de l'os d'origine, après des opérations de délipidation en elles-mêmes classiques, alors que le collagène de structure n'est pratiquement pas attaqué. Le matériau obtenu se distingue, non seulement par ses propriétés à l'emploi, mais également par ses caractéristiques de composition et de structure, de tous les matériaux connus antérieurement pour la même application, en particulier de ceux qui prévoyaient une réincorporation de collagène de peau dans un squelette d'os déminéralisé, ou de ceux qui vise à une restructuration du collagène, car ceci apparaît conduire à un système de cristallisation différent de la cristallisation naturelle.

Dans un mode de mise en oeuvre préféré de l'invention, le procédé ne comporte pas d'opérations préalables de déminéralisation partielle de l'os délipidé. Cependant, on peut aussi utiliser à ce stade les techniques connues de déminéralisation.

Après l'étape essentielle d'extraction suivant l'invention, le procédé comporte avantageusement une étape de lavage ultérieur, en elle-même classique, qui permet d'éliminer ces protéines de la matière osseuse en cours de traitement, sélectivement par rapport au collagène de structure non dénaturé. Dans la mise en oeuvre du procédé selon l'invention, cette étape de lavage est avantageusement effectuée à une température comprise entre 30 °C et 60 °C, et de préférence de l'ordre de 45 °C à 55 °C. On réalise de la sorte, d'une manière bénéfique pour les propriétés finales de la matière à implanter en chirurgie osseuse, un compromis avantageux entre l'élimination souhaitable des protéines et la préservation du collagène sous forme non tannée.

Suivant des caractéristiques secondaires de l'invention, le procédé peut impliquer une répétition de l'étape essentielle d'extraction sélective par l'urée, avantageusement combinée à un autre agent capable de rompre les ponts disulfures tel que le mercapto-éthanol, cette étape étant à chaque fois suivie d'une étape de lavage appropriée.

Par ailleurs, le procédé global peut comporter en outre diverses étapes qui sont en elles-mêmes classiques, et notamment parmi celles-ci, une étape d'extraction par un solvant ionique, constitué en particulier par une solution aqueuse d'un sel tel que le chlorure de sodium.

On notera que dans la mise en oeuvre pratique du procédé selon l'invention, les agents d'extraction utilisés peuvent être aisément choisis parmi des composés ne présentant aucune toxicité vis-à-vis de l'organisme, ce qui n'est pas le cas par exemple du glutaraldéhyde utilisé dans l'art antérieur.

Dans ses modes de mise en oeuvre préférés, le procédé selon l'invention conduit à une matière osseuse dans laquelle les techniques usuelles d'analyse permettent de déceler la présence de collagène de structure, en proportion avantageusement comprise entre 20 et 40 %, et de préférence de l'ordre de 25 à 35 %.

Il est donc clair qu'à l'issue du traitement, le matériau obtenu ne contient pas la totalité du collagène d'origine, dont on sait qu'il représente environ 90 % de la matière organique de l'os sec dégraissé, avec environ 95 % de cette proportion en collagène de type I, caractérisé par une molécule de tropocollagène formée de trois chaînes polypeptiques agencées en hélice comprenant deux chaînes alpha-1 de type I et une chaîne alpha-2 de type I, et environ 5 % de collagène de type III, qui comporte trois chaînes alpha-1 identiques. Néanmoins, seul le collagène aisément extractible en même temps que d'autres protéines est évacué lors des opérations classiques de délipidation préalable et déminéralisation éventuelle, alors que le collagène de structure reste en majeure partie non attaqué quand on dénature par l'urée les autres protéines non éliminées précédemment, essentiellement constituées de protéoglycanes ou glycoprotéines antigéniques.

Or les qualités du matériau obtenu par le procédé de l'invention, notamment son aptitude à l'ostéointégration, mais aussi ses propriétés mécaniques et sa facilité à être façonné, semblent précisément liées à cette présence de collagène d'origine, intégré dans la structure microporeuse, et constitué principalement par du collagène de type I. Il est à remarquer en outre que l'invention conduit en général à un enrichissement relatif du collagène en collagène de type I, jusqu'à une teneur, par exemple, de 97 % de collagène de type I pour 3 % de collagène de type III.

Ceci se constate sur le matériau obtenu, par les techniques d'analyse connues de caractérisation des types de collagènes, qui consistent par exemple soit à observer le collagène par immunofluorescence après l'avoir fait réagir avec des anticorps anti-collagène de type I, soit à le soumettre à l'action du bromure de cyanogène pour obtenir un mélange de peptides caractérisables qui sont séparés en fonction de leurs poids moléculaires et analysés par électrophorèse.

Le plus fréquemment le matériau selon l'invention, obtenu suivant le procédé avec déminéralisation partielle préalable, présente une composition comprise entre les gammes indiquées ci-après (en % en poids) :

| | |
|---|---|
| Eau | 2 - 10 ou inférieur à 10 |
| Lipides | 1-15 ou < 15 |
| Cendres | 40 - 65 |
| Calcium | 10 - 25 |
| Phosphore | 5 - 20 |
| Ca/P | 1 - 2,2 |
| Protéines | 30 - 45 |
| Collagène | 20 - 40 |

Dans le cas préféré où le procédé ne comporte pas de déminéralisation partielle préalable, il présente avantageusement une composition comprise entre les gammes indiquées ci-après :

| | |
|---|---|
| Eau | < 10 |
| Lipides | < 2, soit 0,1-2 |
| Minéraux | 50 - 70 |
| Calcium | 20 - 30 |
| Phosphore | 10 - 17 |
| Ca/P | 1,6 - 2,2 |
| Protéines | 25 - 35 |
| Collagène | 20 - 30 |

On décrira maintenant l'invention plus en détail dans le cadre d'exemples particuliers de mise en oeuvre du procédé et d'utilisations cliniques du matériau qui ne sont nullement limitatifs.

### EXEMPLE I : Préparation du matériau en blocs

On utilise comme matière de départ des blocs d'os préalablement découpés dans des épihyses ou diaphyses d'os de bovidé fraîchement obtenues à l'abattoir. Selon l'usage souhaité, on préfèrera l'os spongieux (épiphyses) ou l'os cortical (diaphyses). Dans le cas présent, où l'on souhaite une bonne résistance à la compression, on effectue plutôt des coupes transversales d'épiphyses, perpendiculaires au réseau trabéculaire.

Ces blocs sont traités d'abord suivant une étape classique de délipidation du tissu osseux, par un mélange chloroforme/méthanol ou éthanol/dichlorométhane, le volume du mélange délipidant étant d'environ 10 volumes pour 1 partie d'os frais de départ (10 l/kg). Cette étape permet d'extraire des lipides libres, des acides gras, des lipoprotéines, associés à la trame osseuse. Elle est effectuée ici de la manière suivante : 400 g de blocs d'os sont traités par un mélange de 4 l de chloroforme-méthanol (2/3-1/3) à une température de 4 °C pendant 24 h, sous agitation.

Dans la suite des opérations et sauf indication contraire, cette proportion de 10 volumes pour une partie d'os de départ sera conservée.

Dans un exemple **I A**, mais non dans un exemple **I B**, les blocs sont ensuite soumis à une étape de déminéralisation, réalisée par contact avec l'acide chlorhydrique 0,6 M à 4 °C pendant 6 h. Cette étape est classique en elle-même, et l'on peut en faire varier les conditions d'après ce qui en a été décrit par exemple dans le brevet français 2 582 517, en utilisant une solution d'acide chlorhydrique de molarité comprise entre 0,1 M et 1 M, ou d'un autre acide minéral ou carboxylique, et/ou un agent organique capable de complexer le calcium de l'os comme l'éthylène-diamine-tétraacétique.

En variante, on peut aussi allonger le temps de contact avec HCl, par exemple à 36 heures. Suivant le temps de traitement, on obtient un matériau d'une dureté plus ou moins grande, adaptée à celle désirée pour l'utilisation future.

Au cours de cette étape de déminéralisation, on enlève donc une partie des ions constitutifs de l'hydroxyapatite liée à la trame collagénique osseuse, rendant cette dernière plus accessible pour les extractions à venir.

L'étape suivante consiste en une extraction par contact avec une solution aqueuse de chlorure de sodium : les blocs sont traités par 4 l de soluté de chlorure de sodium 1 M à la température de 4 °C, sous agitation, pendant 36 heures.

Cette extraction permet d'éliminer de la matière osseuse les composants macromoléculaires les plus solubles ou fraîchement synthétisés. Dans l'extrait on trouve des lipides qui avaient subsisté après les traitements précédents, des protéoglycanes et des collagènes. Cette étape permet de ne pas dénaturer la trame collagénique osseuse tout en extrayant partiellement quelques composants avant d'attaquer par les étapes d'extractions suivantes, les composants matriciels les plus résistants.

On en arrive alors, dans l'exemple I A comme dans l'exemple I B, à l'étape essentielle de l'invention, suivant laquelle on met les blocs d'os en contact avec 4 l d'urée 8 M à la température ambiante (20 °C) pendant 6 heures.

Cette étape permet d'extraire des molécules telles que protéoglycanes et glycoprotéines, y compris les glycoprotéines de structure qui sont les molécules qui présentent la plus forte antigénicité dans l'os, mais sans pour autant dénaturer de trop la trame conjonctive collagénique.

Cette extraction est complétée par une nouvelle extraction à finalité similaire, effectuée à l'aide de 4 l d'une solution de mercapto-éthanol à 0,2 % dans l'urée 8 M à température ambiante pendant 24 h.

A l'issue de l'ensemble de ces deux étapes d'extraction sélective, on se trouve avoir extrait les éléments glycoprotéiques, non collagéniques, ou du moins les avoir dénaturés de manière efficace, empêchant ainsi le matériel à greffer d'être rejeté de par l'antigénicité naturelle de ses glycoprotéines entrant dans la composition macromoléculaire de la trame osseuse.

Les différents solvants et réactifs utilisés au cours des opérations précédentes sont ensuite éliminés par un lavage à l'aide de 4 l d'eau distillée à 55 °C, pendant 24 heures.

Puis vient un traitement par un tampon phosphate 0,4 M à pH = 7,4, qui est effectué sous agitation pendant 48 h à 35 °C. Et cette opération est suivie par un rinçage à l'aide de 2 l d'éthanol, à température ambiante pendant 24 heures.

Un lavage énergique à l'eau pure permet d'enlever, ou plutôt de poursuivre l'extraction des lipides, des protéoglycanes, des collagènes et des glycoprotéines. La température de traitement utilisée permet d'inhiber les activités enzymatiques présentes dans les tissus, à savoir celles issues des protéases endogènes, ou des protéases exogènes d'origine bactérienne par exemple, ainsi que d'autres enzymes lytiques.

Quant à l'extraction au tampon phosphate, elle poursuit l'élimination des composants matriciels et/ou lipidiques qui pourraient être encore accrochés à la trame osseuse. Elle permet également de rééquilibrer le matériel osseux en sels.

Enfin, le lavage dans un bain d'alcool, permet de parfaire l'extraction des composés encore faiblement associés à la trame collagénique, en particulier des lipides résiduels, mais facilite surtout l'opération d'isolement ultérieure.

En effet, les fragments osseux traités suivant l'invention pour obtenir un matériau d'ostéoplastie, sont finalement égouttés, rincés à l'alcool éthylique et séchés en étuve ventilée. Ils peuvent être également congelés et/ou lyophylisés, pour être conservés jusqu'à leur utilisation. Les pièces individuelles peuvent aisément être façonnées pour obtenir les formes propres à être utilisées. C'est ainsi que le matériau peut se présenter sous la forme de blocs parallèlépipédiques de différentes dimensions, de troncs de pyramide, de lamelles, de bouchons centro-médullaires, de disques, etc. Les formes définitives sont alors conditionnées et stérilisées par rayonnement ionisant.

### EXEMPLE II : Etude analytique

Le matériau finalement obtenu a été soumis à des examens microscopiques, biochimiques et chimiques.
a) Examen en microscopie optique, lumière optique et polarisée. Il montre une conservation de la structure du tissu osseux. On retrouve en particulier l'aspect caractéristique du réseau trabéculaire du tissu spongieux. On vérifie en particulier que le collagène se trouve dans sa disposition d'origine ce qui différencie très nettement le matériau selon l'invention des éventuels produits de l'art antérieur qui résulteraient d'un apport de collagène exogène sur un support quelconque.
b) Examen en microscopie de fluorescence. Il permet la caractérisation de la structure lamellaire spécifique, très proche de celle de l'os naturel. la caractérisation du collagène de type I a été réalisée par l'utilisation d'anticorps anti-collagènes type 1 spécifiques.
c) En biochimie, le typage au bromure de cyanogène, confirme que le collagène est bien de type I. On relève la présence de peptides alpha 1 CB7 et alpha 1 CB8. La présence de peptides alpha 1 CB6 confirme la réticulation des chaînes et donc la conservation partielle de la structure spatiale. On note également la présence d'alpha C B3.
   Ces résultats, comparés aux substances de référence, démontrent la présence d'un collagène de type 1 caractérisé.
d) En chimie, une moyenne des analyses effectuées sur quatre lots résultant de la mise en oeuvre du procédé selon l'Exemple I donne les résultats suivants, exprimés en pourcentage en poids par rapport au poids total des matières sèches :

### Exemple I A :

| Dosage | Moyenne % | Valeurs extrêmes |
|---|---|---|
| Perte à la dessication | 6,8 | 6,3 - 7,3 |
| Lipides | 10,8 | 7,4 - 14,0 |
| Cendres | 48,4 | 44,6 - 53 |
| Calcium | 16,8 | 15,5 - 18,0 |
| Phosphore | 11,5 | 8,8 - 13,4 |
| Ca/P | 1,49 | 1,22 - 1,86 |
| Protéines | 36,8 | 35,4 - 38,5 |
| Collagène | 30,2 | 29,5 - 30,7 |

Exemple I B (sur six lots) :

| Dosage | Moyenne % | Valeurs extrêmes |
|---|---|---|
| Eau | 8,9 | 7,3 - 9,7 |
| Lipides | < 0,3 | < 0,3 |
| Cendres | 61,7 | 60 - 65 |
| Calcium | 22,64 | 21,5 -25,3 |
| Phosphore | 11,5 | 10,3 - 12,4 |
| Ca/P | 1,97 | 1,7 - 2,22 |
| Protéines | 29,6 | 28 - 31 |
| Collagène | 27,3 | 25,6 - 29,3 |

La densité apparente moyenne du produit varie entre 0,32 et 0,57 g/cm³, suivant les échantillons, autour d'une valeur moyenne de 0,39 g/cm³.

### EXEMPLE III A : Préparation d'un matériau en poudre

Des fragments prélevés sur des os bovins, sans qu'il soit besoin de se soucier de l'orientation des plans de coupe, sont traités comme il a été décrit dans l'exemple I. Ils sont ensuite réduits en poudre, celle-ci peut être utilisée en tant que telle.

### EXEMPLE III B : Préparation d'un matériau moulé

En variante, à partir de poudres de différentes granulométries, on moule des pièces aux formes désirées à l'aide d'un liant, qui peut être avantageusement d'origine biologique mais aussi éventuellement et selon l'utilisation, un polymère synthétique biodégradable.

Dans le cas particulier du présent exemple, on a utilisé un matériau servant de liant et façonné de manière à permettre la revascularisation tel qu'un polymère biorésorbable de la série des polylactiques dans la proportion de 1 à 10 parties pour 1 partie de poudre d'os. On pourrait aussi utiliser de la fibrine dans la proportion de 1 ou 2 parties pour 100 parties d'os. La granulométrie des poudres varie de 1000 à 250 µ.

Les formes définitives sont alors stérilisées par irradiation et conditionnées sous emballage stérile.

### EXEMPLE IV : Variantes du procédé

On traite des blocs d'os bovin comme dans l'exemple 1, sauf que l'on fait précéder le lavage final à l'alcool, avant ou après lavage à l'eau distillée, par une nouvelle étape de délipidation par un mélange de chloroforme et de méthanol, ou un mélange d'éthanol et de dichlorométhane.

Cette étape permet de parfaire la délipidation de la trame osseuse conjonctive et donc d'éliminer les substances lipidiques, acides gras ou lipoprotéines qui demeurent encore liés à la trame osseuse. Une délipidation ainsi poussée permet par exemple l'obtention dans les meilleures conditions d'une poudre d'os selon l'exemple III dont la composition en lipides résiduels est avantageusement ramenée à un taux inférieur à 5 %.

De même, il peut être opportun de rajouter deux étapes supplémentaires de délipidation, ceci permet alors d'obtenir un produit dont le taux de lipides résiduels devient tout à fait négligeable, à savoir de l'ordre de 1 ou inférieur à 1 %.

Bien entendu, dans ces conditions les pourcentages relatifs des autres constituant s'en trouvent modifiés.

On a par ailleurs fait varier les conditions des différentes étapes de traitement, notamment en concentrations, temps de contact et températures, en les appliquant à des blocs dont les trois dimensions variaient entre 8 et 30 centimètres. Les conditions utilisées se situaient dans les limites suivantes :
- Suppression de l'étape d'extraction par un solvant ionique, qui dans d'autres cas est effectuée au moyen d'une solution de NaCl 0,5 à 2 M, à une température de 2 à 5 °C pendant 12 à 36 heures.
- Solution aqueuse d'urée de concentration comprise entre 5 et 10 M.
- Solution aqueuse de mercapto-éthanol dans l'urée contenant de 0,1 à 0,5 % de mercapto-éthanol en volume.
- Temps d'extraction sélective par cette solution compris entre 12 et 36 heures à la température ambiante.

On a ainsi obtenu des matériaux satisfaisants en ostéoplastie dont les résultats d'analyse se situent dans les gammes ci-après (% en poids) :

| | |
|---|---|
| Cendres | 40 à 70, notamment 60 à 65 % |
| Lipides | 0,5 à 2 % |
| Perte à la dessication | 7 à 10 % |
| Protéines (Kjeldahl) | 25 à 45 % |
| Calcium | 10 à 30 % (10 à 20 ou 20 à 30) |
| Phosphore | 10 à 17 % |
| Rapport Ca/P H | 1 à 2,2 (1 à 1,3 ou 1,6 à 2,2 |
| Collagène | 33 à 38 % |
| (dosage du collagène selon l'hydroxyproline) | |

Les mêmes résultats sont obtenus dans les variantes de l'invention qui consiste à passer par l'intermédiaire d'une poudre comme il est décrit à l'exemple III.

### EXEMPLE V : Observation clinique sur un chat mâle castré âgé d'une année

Cet animal a été victime d'un accident (choc traumatique par automobile) qui a entraîné une fracture comminutive du fémur droit au niveau de la région trochantérienne et métaphysaire proximale). En utilisant le matériau d'ostéoplastie selon l'invention, il a été opéré huit jours après le traumatisme par la technique suivante : abord classique du foyer fracturaire, encastrage d'un bloc préparé selon l'exemple I dans le massif trochantérien reconstitué et synthèse par reconstruction de la diaphyse fémorale à l'aide du fixateur externe Pérot de petite taille (broches transfixantes et filetées d'un diamètre de 2 mm).

L'animal a retrouvé une fonction de marche, course et saut tout à fait normale. Les radiographies effectuées à 60 et 90 jours montrent une reconstruction osseuse satisfaisante et une prise de la greffe. Ceci a permis d'éviter les complications d'une fracture ayant pu aboutir à une amputation du membre en cas de non consolidation.

### EXEMPLE VI : Chienne berger allemand

Cet animal a été victime d'un accident de la circulation ayant entraîné une fracture diaphysaire comminutive avec esquille. Il a été opéré rapidement et il a été procédé à la mise en place d'un greffon hétérologue, préparé à partir d'un bloc d'os bovin traité comme dans l'exemple I. Le greffon était sculpté dans ce bloc de telle façon que l'on puisse obtenir un montage par mortaise au niveau des abouts fémoraux diaphysaires. La solidification du montage d'ostéosynthèse a été réalisée par apposition d'une plaque droite Pérot et vissage à l'aide de vis d'un diamètre de 3,5 mm sur la diaphyse fémorale.

L'animal a retrouvé son appui dès les jours suivants. Les contrôles radiographiques effectués à trente et soixante jours montrent la stabilité du montage et la prise du greffon.

### EXEMPLE VII : Utilisation du matériau selon l'invention sous forme pulvérisée pour la résection apicale sur l'incisive latérale supérieure gauche d'une patiente.

L'intervention a été pratiquée suivant une technique classique : incision semi-lunaire avec exérèse du kyste et stérilisation du site au laser CO2. La mise en place du matériau selon l'invention, sous forme pulvérisée, obtenu selon l'Exemple III, a été effectuée en le mélangeant à du sérum physiologique stérile et en le comprimant à l'intérieur de la géode osseuse sanguinolente. Le lambeau gingival est ensuite remis en place et suturé et une radiographie de contrôle est faite.

L'observation à 8 jours permet de constater un excellent début de cicatrisation. A 8 jours l'image radiographique est d'une qualité bien meilleure que celles obtenues avec les autres matériaux de comblement classiquement utilisés : la radio-opacité du matériau selon l'invention se rapproche de celle de l'os alvéolaire humain.

A 15 jours et à 1 mois, les radiographies confirment ces résultats. La cicatrisation est parfaite et aucun phénomène de rejet ne s'est manifesté.

L'aspect radiographique permet d'affirmer qu'il y a "intégration" de la poudre, ce qui d'après l'expérience du praticien ayant effectué cette opération est tout à fait exceptionnel par rapport à ce qu'il a observé avec les autres produits de comblement connus sur le marché.

### EXEMPLE VIII : Utilisation du matériau selon l'invention pour l'assainissement de deux poches parodontales chez une patiente de 50 ans.

Après avoir récliné un lambeau qui s'étend de l'incisive centrale supérieure gauche à la 2ème molaire supérieure gauche, on procède à la stérilisation de la partie osseuse et radiculaire des poches par un détartrage profond, curetage et surfaçage radiculaire.

Le tissu de granulation au niveau gingival sur le lambeau et à l'intérieur des poches a été volatilisé à l'aide d'un laser CO2, puis le périoste et les poches ont été rincés avec du sérum physiologique.

On a utilisé deux blocs du matériau préparé selon l'Exemple I. Ces blocs stériles sont taillés aux dimensions voulues dans des conditions rigoureusement stériles. Ces deux blocs ont été placés en force, de façon à établir un contact étroit et servir de moyen de contention. Ce travail achevé, il a été procédé à la remise en place du lambeau et à sa suture.

Huit jours après la dépose du pansement chirurgical et après que l'on ait enlevé les points de suture et nettoyé le site d'intervention, on a pu constater que certains points de suture (2) avaient lâché et que le recouvrement des deux blocs n'était pas parfait puisqu'ils étaient encore un peu visibles. Malgré cela l'état de cicatrisation était satisfaisant et, chose extraordinaire, une néo-gencive telle qu'il s'en développe normalement au niveau du périoste quand celui-ci a été mis à nu, se développe au-dessus des blocs. Il y a donc "reconnaissance" du matériau selon l'invention, par la néogencive. Ceci est confirmé par le contrôle radiographique.

A 15 jours, les deux blocs du matériau selon l'invention étaient recouverts entièrement par le lambeau de transfert et par de la néogencive au niveau des zones découvertes.

Ceci confirme que par sa "neutralité" ou par sa structure proche de celle de l'os alvéolaire humain, le matériau selon l'invention s'intègre parfaitement et se recouvre d'une néogencive comme à partir du périoste.

La dernière radio à un mois permet de confirmer que les deux blocs sont intégrés parfaitement dans leur site. Aucun phénomène de rejet n'est constaté. D'autre part, les dents ne sont plus mobiles et l'aspect gingival est redevenu normal, ce qui est exceptionnel étant donné les problèmes de suture en post-opératoire.

## Revendications

1. Procédé de fabrication d'un matériau pour ostéoplastie caractérisé en ce qu'on part d'un tissu osseux d'origine humaine ou animale qui est ensuite soumis à un traitement qui comporte au moins une étape d'extraction et/ou dénaturation sélective des protéines de structure non collagéniques, réalisée au moyen d'un agent d'extraction sélectif à base d'urée afin d'obtenir ainsi un matériau pour l'ostéoplastie qui conserve la structure osseuse naturelle d'origine.

2. Procédé suivant la revendication 1, caractérisé en ce que l'urée est sous forme d'une solution aqueuse, sous une concentration comprise entre 2 et 10 M, et de préférence entre 5 et 8 M.

3. Procédé suivant la revendication 1 ou 2, caractérisé en ce que préalablement à ladite étape d'extraction sélective, ladite matière osseuse est soumise à un traitement de délipidation en soi connu.

4. Procédé suivant la revendication 3, caractérisé en ce que la délipidation est effectuée par contact avec un mélange chloroforme/méthanol ou éthanol/dichlorométhane, en proportion d'environ 10 volumes pour 1 partie d'os (10 l/kg).

5. Procédé suivant la revendication 3 ou 4, caractérisé en ce que préalablement à ladite étape d'extraction sélective et après ladite étape de délipidation, ladite matière osseuse est soumise à un traitement de déminéralisation en soi connu.

6. Procédé suivant la revendication 5, caractérisé en ce que la déminéralisation est effectuée au moyen d'une solution d'acide chlorhydrique de molarité comprise entre 0,1 M et 1 M.

7. Procédé suivant l'une quelconque des revendications 1 à 6, caractérisé en ce que ladite extraction sélective est suivie par une étape d'extraction sélective complémentaire au moyen d'une solution aqueuse d'urée additionnée de mercapto-éthanol, présent de préférence à une concentration comprise entre 0,1 et 0,5 % en volume.

8. Procédé suivant l'une quelconque des revendications 1 à 7, caractérisé en ce que ladite extraction sélective est précédée par une étape d'extraction des constituants solubles dans un solvant ionique tel que le chorure de sodium.

9. Procédé suivant l'une quelconque des revendications 1 à 7, caractérisé en ce que postérieurement à ladite extraction sélective, il comporte au moins une opération de lavage entraînant les glycoprotéines de structure dénaturées.

10. Procédé suivant la revendication 9, caractérisé en ce que ladite opération de lavage est effectuée au moyen d'eau distillée à une température comprise entre 30 °C et 60 °C, et de préférence de l'ordre de 45 °C à 55 °C.

11. Matériau d'ostéoplastie obtenu par un procédé selon l'une quelconque des revendications 1 à 10, caractérisé par la conservation de la structure osseuse naturelle d'origine, intégrant du collagène de type I non dénaturé.

12. Matériau suivant la revendication 11, caractérisé en ce qu'il contient du collagène de structure, en proportion comprise entre 20 et 40 %, et notamment de l'ordre de 25 à 35 %.

13. Matériau selon les revendications 11 et 12, caractérisé en ce qu'il comporte en analyse de matières sèches une proportion de lipides inférieure à 15 %, une proportion de protéines comprise entre 25 et 45 %, une proportion de calcium de 10 à 30 %, une proportion de phosphore de 5 à 20 %, une teneur en eau inférieure à 10 %, le rapport Ca/P étant avantageusement de 1 à 2,2.

14. Matériau selon l'une des revendications 11 à 13, caractérisé en ce qu'il se présente sous la forme de blocs parallélépipédiques, de troncs de pyramide, de lamelles, de bouchons centro-médullaires, de disques ou de poudre ou encore de poudre amalgamée à l'aide d'un liant qui peut être avantageusement d'origine biologique tel que la fibrine ou d'origine synthétique tel que par exemple un polymère synthétique biodégradable.

15. Matériau selon l'une quelconque des revendications 11 à 14, caractérisé en ce qu'il est obtenu par le procédé selon l'une quelconque des revendications 1 à 10.

## Claims

1. Method for manufacturing a material for osteoplasty characterized in that one proceeds from bone tissue of human or animal origin which is then subjected to a treatment that involves at least one step of selective extraction and/or denaturation of the non-collagenous structural proteins, using a selective extraction agent having a base of urea, to obtain thereby a material for osteoplasty that retains the original natural bone structure.

2. Method in accordance with claim 1, characterized in that the urea is in the form of an aqueous solution at a concentration within the range of 2 to 10 M and preferably 5 to 8 M.

3. Method in accordance with claim 1 or claim 2, characterized in that, prior to said selective extraction step, said bone material is subjected to a delipidation treatment known per se.

4. Method in accordance with claim 3, characterized in that delipidation is carried out by contact with a mixture of chloroform and methanol or ethanol and dichloromethane in a proportion of approximately 10 volumes to 1 part of bone (10/1kg).

5. Method in accordance with claim 3 or claim 4, characterized in that, prior to said selective extraction step and after said delipidation step, said bone material is subjected to a demineralization treatment known per se.

6. Method in accordance with claim 5, characterized in that demineralization is carrried out by means of a hydrochloric acid solution having a molar concentration within the range of 0.1 M to 1M.

7. Method in accordance with any one of claims 1 to 6, characterized in that said selective extraction is followed by a step involving complementary selective extraction by means of an aqueaous solution of urea containing an addition of mercaptoethanol which is preferably present in a concentration within the range of 0.1 to 0.5 volume %.

8. Method in accordance with any one of claims 1 to 7, characterized in that said selective extraction is preceded by a step involving extraction of the soluble constituents into an ionic solvent such as sodium chloride.

9. Method in accordance with any one of claims 1 to 7, characterized in that, after completion of said selective extraction, said method involves at least one washing operation which entrains the denatured structural glycroproteins.

10. Method in accordance with claim 9, characterized in that said washing operation is carried out by means of distilled water at a temperature within the range of 30°C to 60°C and preferably of the order of 45°C to 55°C.

11. Osteoplasty material obtained by a method in accordance with any one of claims 1 to 10, characterized in that said material retains the original and natural bone structure integrating non-denatured type I collagen.

12. Material in accordance with claim 11, characterized in that it contains structural collagen in a proportion within the range of 20 to 40 % and especially of the order of 25 to 35 %.

13. Material in accordance with claim 11 and claim 12, characterized in that said material is found by dry analysis to contain a proportion of lipids lower than 15 %, a proportion of proteins within the range of 25 to 45 %, a proportion of calcium of 10 to 30 %, a proportion of phosphorus of 5 to 20 %, a water content lower than 10 %, the ratio Ca/P being advantageously from 1 to 2.2.

14. Material in accordance with any one of claims 11 to 13, characterized in that said material is provided in the form of parallelepipedal blocks, pyramidal frustums, lamellae, centro-medullary end-caps, disks or powder, or in the form of powder amalgamated with a binder which can advantageously be of biological origin such as fibrin or of synthetic origin such as for example a biodegradable synthetic polymer.

15. Material in accordance with any one of claims 11 to 14, characterized in that said material is obtained by the method in accordance with any one of claims 1 to 10.

## Patentansprüche

1. Verfahren zur Herstellung eines Materials für die Osteoplastik, dadurch gekennzeichnet, daß man von einem Knochengewebe menschlichen oder tierischen Ursprungs ausgeht, welches dann einer Behandlung unterworfen wird, die zumindest eine selektive Extraktions- und/oder Denaturierungsstufe für Proteine nicht-kollagener Struktur umfaßt, welche mit Hilfe eines selektiven Extraktionsmittels auf Harnstoffbasis ausgeführt wird, um so ein Material für die Osteoplastik zu erhalten, welches die ursprüngliche, natürliche Knochenstruktur konserviert.

2. Verfahren nach Anspruch 1, dadurch gekennzeichnet, daß der Harnstoff in Form einer wäßrigen Lösung in einer Konzentration zwischen 2 und 10 M, vorzugsweise zwischen 5 und 8 M, verwendet wird.

3. Verfahren nach Anspruch 1 oder 2, dadurch gekennzeichnet, daß vor der besagten selektiven Extraktionsstufe die besagte Knochensubstanz einer an sich bekannten Behandlung zur Entfettung unterworfen wird.

4. Verfahren nach Anspruch 3, dadurch gekennzeichnet, daß man die Entfettung durchführt, indem man mit einer Mischung aus Chloroform/Methanol oder Ethanol/Dichlormethan in einem Verhältnis von etwa 10 Volumina zu einem Teil Knochen (10 l/kg) behandelt.

5. Verfahren nach Anspruch 3 oder 4, dadurch gekennzeichnet, daß die besagte Knochensubstanz vor der besagten selektiven Extraktionsstufe und nach der besagten Entfettungsstufe einer an sich bekannten Behandlung zur Demineralisierung unterworfen wird.

6. Verfahren nach Anspruch 5, dadurch gekennzeichnet, daß die Demineralisierung mit Hilfe einer Lösung von Chlorwasserstoffsäure mit einer Molarität zwischen 0,1 M und 1 M durchgeführt wird.

7. Verfahren nach einem der Ansprüche 1 bis 6, dadurch gekennzeichnet, daß der besagten selektiven Extraktion eine komplementäre selektive Extraktionsstufe mit Hilfe einer wäßrigen Harnstofflösung mit zugesetztem Mercaptoethanol in einer bevorzugten Konzentration zwischen 0,1 und 0,5 Vol.-% folgt.

8. Verfahren nach einem der Ansprüche 1 bis 7, dadurch gekennzeichnet, daß der besagten selektiven Extraktion eine Extraktionsstufe von löslichen Bestandteilen in einem ionischen Lösungsmittel, wie Natriumchlorid, vorausgeht.

9. Verfahren nach einem der Ansprüche 1 bis 7, dadurch gekennzeichnet, daß es im Anschluß an die besagte selektive Extraktion mindestens einen Spülvorgang beinhaltet, bei dem die Glycoproteine mit denaturierter Struktur entfernt werden.

10. Verfahren nach Anspruch 9, dadurch gekennzeichnet, daß der besagte Spülvorgang mit Hilfe von destilliertem Wasser bei einer Temperatur zwischen 30°C und 60°C, vorzugsweise in der Größenordnung von 45°C bis 55°C, durchgeführt wird.

11. Osteoplastisches Material, erhalten durch ein Verfahren nach einem der Ansprüche 1 bis 10, gekennzeichnet durch die Konservierung der ursprünglichen natürlichen Knochenstruktur, wobei es nicht-denaturiertes Kollagen vom Typ I enthält.

12. Material nach Anspruch 11, dadurch gekennzeichnet, daß es Strukturkollagen in einem Anteil zwischen 20 und 40 % und insbesondere in der Größenordnung von 25 bis 35 % enthält.

13. Material nach den Ansprüchen 11 und 12, dadurch gekennzeichnet, daß es in der Trockensubstanzanalyse einen Lipidanteil von weniger als 15 %, einen Proteinanteil zwischen 25 und 45 %, einen Calciumanteil von 10 bis 30 %, einen Phosphoranteil von 5 bis 20 % und einen Wassergehalt von weniger als 10 % aufweist, wobei das Verhältnis Ca/P vorteilhafterweise 1 bis 2,2 beträgt.

14. Material nach einem der Ansprüche 11 bis 13, dadurch gekennzeichnet, daß es mit Hilfe eines Bindemittels, welches vorteilhafterweise biologischen Ursprungs, wie das Fibrin, oder synthetischen Ursprungs, wie z. B. ein biologisch abbaubares, synthetisches Polymer, sein kann, in Form von parallelepipedischen Blöcken, Pyramidenstümpfen, Lamellen, zentromedullären Stopfen, Scheiben oder Pulvern oder sogar amalgamierten Pulvern vorliegt.

15. Material nach einem der Ansprüche 11 bis 14, dadurch gekennzeichnet, daß es durch ein Verfahren nach einem der Ansprüche 1 bis 10 erhalten wird.
